# EUROPÄISCHE PATENTANMELDUNG

(11) **EP 0 806 208 A2**
(43) Veröffentlichungstag der Anmeldung: **12.11.1997**
(21) Anmeldenummer: 97107316.8
(22) Anmeldetag: 02.05.1997
(51) Int. Cl.: A61K 35/78

(54) **Verwendung von Bärlauch zur Herstellung eines Arzneimittels zur Behandlung und/oder Prophylaxe von Mykosen**

(30) Priorität: 10.05.1996 DE 19618930
(71) Anmelder: PANDALIS, Georgios, D-49219 Glandorf (DE)
(72) Erfinder: PANDALIS, Georgios, D-49219 Glandorf (DE)
(74) Vertreter: Grünecker, Kinkeldey, Stockmair & Schwanhäusser Anwaltssozietät

(57) **Zusammenfassung**

Die Erfindung betrifft die Verwendung von Allium ursinum L. (Bärlauch). Es wurde gefunden, daß Bärlauch zur Bekämpfung von Mykosen, insbesondere von enteralen Mykosen, verwendet werden kann. Vorzugsweise können mit Bärlauch durch Candida hervorgerufene Mykosen behandelt werden.

## Beschreibung

Die Erfindung betrifft die Verwendung von Allium ursinum L. (Bärlauch).

Allium ursinum L. (Bärlauch, Bärenlauch, wilder Knoblauch) gehört, wie der Knoblauch, zur Familie der Liliaceae. Die Pflanze ist u.a. in fast ganz Europa beheimatet und wird auch als Heil-, Gewürz- und Gemusepflanze kultiviert. Für Arzneimittel werden die Bärlauchzwiebel (Allii ursini bulbus), das Bärlauchkraut (Allii ursini herba) sowie die ganze, frische, zur Blütezeit gesammelte Pflanze für homäopathische Zwecke verwendet. Die Inhaltsstoffe der Zwiebel und des Krautes sind als identisch anzusehen. (Siehe Hagers Handbuch der Pharmazeutischen Praxis, 4. Band, 5. Auflage, Springer-Verlag, im Druck).

In der Bärlauchzwiebel lassen sich abhängig von Extraktionsmedium und -verfahren unterschiedliche Muster an genuinen und nicht genuin enthaltenden Inhaltsstoffen nachweisen. Dazu gehören Cysteinsulfoxide, wie S-Methyl-L-(+)-cysteinsulfoxid und S-Allyl-L-(+)-cysteinsulfoxid (Alliin) und Thiosulfinate, die durch Fermentation aus den Cysteinsulfoxiden gebildet werden. Beispiele für die Thiosulfinate sind Allylmethylthiosulfinat bzw. Methylallylthiosulfinat, Diallylthiosulfinat (Allicin) und Dimethylthiosulfinat. Weitere, nicht genuin in der Bärlauchzwiebel enthaltene Stoffe sind Dithiine, wie Vinyldithiine, Ajoen (4,5,9-Trithiododeca-1,6-11-trien-9-oxid), das durch Selbstkondensation von Allicin entsteht, sowie Ajoenhomologe. Ferner werden als wasserdampfflüchtige Bestandteile (12 % bezogen auf die frische Bärlauchzwiebel) Methylallyltrisulfid, das den Hauptbestandteil des Wasserdampfdestillats bildet, sowie geringe Mengen an Diallyldisulfid erhalten.

Diese Stoffe sind ebenfalls nicht genuin in der Bärlauchzwiebel enthalten. Weitere, genuin in der Bärlauchzwiebel enthaltene Stoffe sind beispielsweise die freien Aminosäuren L-Arginin, L-Asparagin, L-Asparaginsäure, L-Glutamin, L-Glutaminsäure, L-Glycin, L-Threonin und L-Alanin.

Die frische Zwiebel enthält nach der Fermentation etwa 0,05 bis 0,12 % Allicin.

Untersuchte Wirkungen der Bärlauchzwiebel sind eine Lipoxygenase- und Cyclooxygenasehemmung, sowie eine Hemmung im PAF-Thrombozytenaggregationstest.

Frische Bärlauchblätter enthalten nach der Fermentation etwa 0,005 % Allicin, getrocknete etwa 0,07 %. Ferner enthalten frische Bärlauchblätter etwa 0,007 % wasserdampfflüchtige Bestandteile, die den wasserdampfflüchtigen Bestandteilen der Bärlauchzwiebel entsprechen.

Es ist bekannt, Bärlauchblätter und -zwiebeln, ähnlich wie Allium sativum (Knoblauch), bei Magen-Darm-Störungen, Gärungsdyspepsien, Flatuleszenz, gegen Bluthochdruck und Arteriosklerose einzusetzen. Äußerlich finden sie Anwendung bei Hautausschlägen.

Die Urtinktur aus ganzen, frischen, zu Beginn der Blütezeit gesammelten Bärlauchpflanzen HAB1 ist eine goldgelbe Flüssigkeit mit Geruch und Geschmack nach Knoblauch HAB1. Die Anwendungsgebiete entsprechen dem homöopathischen Arzneimittelbild. Dazu gehört beispielsweise Verdauungsschwäche.

Ein Einsatz von Bärlauch in der Schulmedizin erfolgt nicht.

Infektionen durch Pilze (Fadenpilze, Hefen, Schimmelpilze und Aktinomyceten) können zu Mykosen führen. Man unterscheidet endogene Mykosen (z.B. enterale Mykosen) und exogene Mykosen (Dermatomykosen). Die wichtigsten Human-Mykosen und ihre Erreger sind in der folgenden Tabelle 1 angeführt.

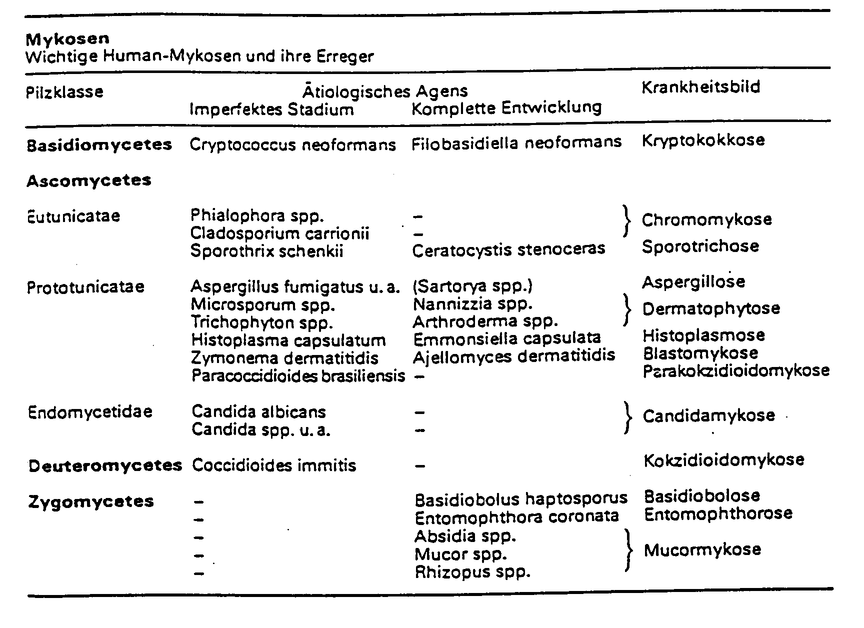

Unter den endogenen Mykosen sind die Candida-Mykosen (Soor-Mykosen, Candidosen) am häufigsten. Als Candida-Mykosen werden Candida-Pilzerkrankungen zusammengefaßt, wobei wiederum vier große Gruppen unterschieden werden: 1. Bronchopulmolare Mykosen; 2. Haut-Mykosen; 3. Schleimhaut-Mykosen; 4. System-Mykosen. (Siehe Pschyrembel Klinisches Wörterbuch, 255. Auflage, de Gruyter, Berlin, 1986).

Candida stellt eine artenreiche Form-Gattung in der Familie Cryptococcaceae der asporogenen Hefen (Deuteromycetes = Fungi imperfecti) dar. (Siehe Römpp Lexikon Biotechnologie, Thieme, Stuttgart, 1992). Für die medizinische Mykologie haben die folgenden Arten Bedeutung: C. albicans, C. tropicalis, C. pseudotropicalis, C. krusei, C. parapsilosis, C. stellatoidea und C. guilliermondii. Der wichtigste Vertreter ist Candida albicans, dünnwandige, gram-positive, kapsellose, nicht sporenbildende Hefen von ovaler bis rundlicher Form, die sich durch Sprossung (Blastosporen, Sproßzellen) vermehren, z.T. aber auch als fadenartige Zellen auftreten, wobei ein sog. Pseudomycel entsteht. Dieser Sproßpilz bekommt unter besonderen Bedingungen Krankheitswert, z. B. nach Einnahme von Antibiotika, oralen Kontrazeptiva, Zytostatika, bei Adipositas, Diabetes mellitus, Gravidität, schweren Allgemeinerkrankungen wie Leukämie, Karzinom, bei Intertrigo und bei langfristigem Arbeiten in feuchtem Millieu. (Siehe Pschyrembel Klinisches Wörterbuch, 255. Auflage, de Gruyter, Berlin, 1986).

Durch Schimmelpilzbefall im Darm und insbesondere enteralem Candida-Befall können Darmstörungen verursacht werden, die auf Milieu-Veränderungen im Darm zurückzuführen sind.

Bei der Behandlung von Pilzerkrankungen kommen verschiedene Antimykotika zum Einsatz.

Aufgabe der vorliegenden Erfindung ist es, Pilzinfektionen mit einem pflanzlichen Präparat zu bekämpfen, das keine Nebenwirkungen zeigt und eine hohe Akzeptanz in der Bevölkerung genießt.

Diese Aufgabe wird durch die Verwendung von Allium ursinum L. (Bärlauch) zur Bekämpfung von Mykosen gelöst.

Bärlauch kann sowohl zur Behandlung als auch zur Prophylaxe von Mykosen verwendet werden.

Es wurde überraschenderweise gefunden, daß die Einnahme von Bärlauch-Präparaten Beschwerden, die durch Pilze, insbesondere durch Schimmelpilze und Candida, hervorgerufen werden, lindern bzw. beseitigen. Dabei werden keine nachteiligen Nebenwirkungen beobachtet. Vor allem Darmstörungen, denen insbesondere eine Störung der Darmflora zugrundeliegen, können mit Bärlauch-Präparaten erfolgreich behandelt werden. Als pflanzliches Präparat genießen Bärlauch-Präparate gegenüber synthetischen Antimykotika eine hohe Akzeptanz in der Bevölkerung.

Bei einem Befall durch Candida albicans kann eine Verminderung der koloniebildenden Einheiten (KBE) um 1 bis 3 Zehnerpotenzen beobachtet werden. Weiterhin führt die Einnahme von Bärlauch zu einer Verbesserung der Kolonisationsresistenz. Unter Kolonisationsresistenz versteht man den Zustand der Integrität des intestinalen Mukosablockes (wandständige Flora, Schleim, Antibody-painting, Schleimhaut und darmschleimhautassoziiertes Immunsystem). Zur wandständigen Flora mit besonders innigem Kontakt gehören: Lactobacillus spp., Bacteroides spp., Enterococcus spp., Bifidobacterium spp.

Vorzugsweise kann Bärlauch zur Bekämpfung von Pilzerkrankungen verwendet werden, die durch Hefen, insbesondere durch Hefen der Gattung Candida, hervorgerufen werden. Erfindungsgemäß wird Bärlauch insbesondere zur Behandlung von Mykosen, denen eine Infektion durch Candida albicans, Candida tropicalis und/oder Candida glabrata zugrundeliegt, verwendet.

Erfindungsgemäß wird Bärlauch bevorzugt in Form üblicher pharmazeutischer Zubereitungen, wie Lösungen, Konzentrate, Tabletten oder Kapseln oral verabreicht. Nach erfolgter klinischer Prüfung wird die benötige Dosierung der Wirkstoffe in Abhängigkeit von üblichen Faktoren, wie der Natur und Schwere der Erkrankung und dem Körpergewicht des Patienten, vom Arzt festgelegt.

Die folgenden Falldarstellungen belegen die Antipilzwirkung von Bärlauch:

### Falldarstellung I

W.F., männlich, 40 Jahre
*Beschwerden:* gehäuft Infekte, vermehrt LWS-Beschwerden mit Ausstrahlung in beide Knie, vermehrt G-I-Beschwerden nach Süßigkeiten, Müdigkeit, teils Diarrhoen, Cephalgien.
*Befunde:* Darm: Candida albicans 10³ KBE, Enterococcen < 10⁴ (Norm 10⁶ - 10⁷); Lactobacillus spp. 6 x 10³; zell. Immunstatus: Dominante Immunsuppression, verringerte Absolutzellzahlen
*Therapie:* basierend auf Frischblatt-Granulat Kapseln Bärlauch 3 x 2 a.c.
*Ergebnis:* Keine Diarrhoen, keine Cephalgien, WS-Beschwerden wesentlich weniger, Infektneigung wesentlich verringert, aber noch nicht vollständig behoben.

### Falldarstellung II

R.B., weiblich, 53 Jahre
*Beschwerden:* chronischer Husten nach "Grippe" mit Angina. Kratzen, Kitzeln im Hals, teils Diarrhoen, vermehrt Meteorismus
*Befunde:* Darm: Candida albicans 1 x 10⁴ KBE, Enterococcen 5 x 10⁴ (Norm 10⁶ - 10⁷), pH-Wert 7,5; zell. Immunstatus: Dominante Hyperergie, Ratio 3,1, Absolutzellzahlen unterster Normalbereich.
*Therapie:* basierend auf Frischblatt-Granulat Kapseln Bärlauch 3 x 2 a.c.
*Ergebnis:* Atemwegserkrankung behoben. Restliche Beschwerden ebenso. Candida albicans 3 x 10², pH-Wert 6,5, Enterococcen 3 x 10⁵.

### Falldarstellung III

W.G., weiblich, 54 Jahre
*Beschwerden:* Bei vermehrten Infekten gehäuft Antibiotikatherapien, dann rezid Diarrhoen, stark gehäufte Infekte, progrediente Depressionszustände, Erschöpfungszustände, Darmfunktionsstörungen m. Obstipation, Diarrhoen. Im Jahr 95 bei diversen Kontrollen deutliche Veränderungen an der Darmflora, teils mit Proteolyten, jedoch vermehrt Schimmelpilze (bis 10⁶ KBE).
*Befunde:* Darm: Candida albicans 2 x 10⁵ KBE, Enterococcen 2 x 10⁵ (Norm 10⁶ - 10⁷); Schimmelpilze 3 x 10³ (vorherige Kontrolle 6 x 10⁶), Clostridium spp. 1 x 10⁶. Keine Hinweise auf Entzündungen der Darmschleimhaut.
*Therapie:* basierend auf Frischblatt-Granulat Kapseln Bärlauch 3 x 2 a.c.
*Ergebnis:* Gesamtzustand "so gut wie schon lange nicht mehr". Candida albicans 1 x 10³, Schimmelpilze 4 x 10², gleichzeitig Anstieg von Bifidobacterium und Enterococcus spp., Clostridium < 10⁵ KBE.

### Falldarstellung IV

F.R., weiblich, 41 Jahre
*Beschwerden:* 4/94 Iritis mit begleitenden rheumatoiden Gelenkbeschwerden. Rezidiv 9/95. Lymphknotenschwellungen, teils dolent.
*Befunde:* Darm: Candida albicans 1 x 10⁵ KBE, Enterococcen < 10⁴ (Norm 10⁶ - 10⁷). Bifidobacterium spp. 4 x 10⁷ KBE, pH 7,5
*Therapie:* basierend auf Frischblatt-Granulat Kapseln Bärlauch 3 x 2 a.c.
*Ergebnis:* Subjektiver Gesamtzustand wesentlich gebessert, weniger Beschwerden durch Gelenke. Candida albicans 2 x 10², Bifidobacterium 2 x 10⁹, Enterococcus spp. < 10⁴ KBE, pH 6,0

### Falldarstellung V:

N.K., weiblich, 43 Jahre
*Beschwerden:* 6 XII 95 St. p. Bartholinitis, Gyn.: ausgeprägte Dysplasie, Pap Iva, aktueller Infekt HNO, vermehrte psycho-vegetative Belastung, vermehrt Stress.
*Befunde:* Candida albicans 1 x 10³, Schimmelpilze 2 x 10⁴, IgA fäc < 0,1. NB.: Chronische Belastung durch vermehrtes Rauchen, dazu früher 10 Jahre Pille.
*Therapie:* basierend auf Frischblatt-Granulat Kapseln Bärlauch 3 x 2 a.c.
*Ergebnis:* Klinik: 12.2.96 Pap IIId, Candida albicans 3 x 10², Schimmelpilze < 10², IgA fäc 0,4. 22.2.96 Pap II. Allgemeinzustand wesentlich besser. Immunstatus noch ausständig.

## Patentansprüche

1. Verwendung von Allium ursinum L. (Bärlauch) zur Herstellung eines Arzneimittels zur Behandlung und/oder Prophylaxe von Mykosen.

2. Verwendung nach Anspruch 1, dadurch gekennzeichnet, daß es sich um eine ente rale Mykose handelt.

3. Verwendung nach Anspruch 1 oder 2, dadurch gekennzeichnet, daß es sich um eine durch Hefen hervorgerufene Mykose handelt.

4. Verwendung nach Anspruch 3, dadurch gekennzeichnet, daß es sich um eine durch Candida hervorgerufene Mykose handelt.

5. Verwendung nach Anspruch 4, dadurch gekennzeichnet, daß es sich um eine durch Candida albicans, Candida tropicalis und/oder Candida glabrata hervorgerufene Mykose handelt.

6. Verwendung nach einem der vorstehenden Ansprüche, dadurch gekennzeichnet, daß Allium ursinum L. für eine orale Verabreichung formuliert ist.
